# EUROPEAN PATENT APPLICATION

(11) **EP 4 588 937 A1**
(43) Date of publication of application: **23.07.2025**
(21) Application number: 23864825.7
(22) Date of filing: 18.09.2023
(51) Int. Cl.: C07K 16/18, A61K 39/395, A61K 38/17, A61P 9/04

(54) **USE OF NKG2D-BASED CELL ADAPTER MOLECULE IN REMOVAL OF AGING CELLS**

(30) Priority: 16.09.2022 CN 202211132057
(71) Applicant: West China Hospital of Sichuan University, Chengdu, Sichuan 610041 (CN)
(72) Inventor: ZHAO, Xudong, Chengdu, Sichuan 610041 (CN); YANG, Dong, Chengdu, Sichuan 610041 (CN)
(74) Representative: Santoro, Sofia
(86) International application number: PCT/CN2023/119470
(87) International publication number: WO 2024/056097

(57) **Abstract**

Provided is the use of NKG2D-CD3 and NKG2D-CD16 cell adapter molecules in the removal of aging cells and the treatment of aging-cell-accumulation-related diseases, wherein the cell adapter molecules comprise an NKG2D extracellular domain, a connection segment, and a CD3 or CD16 binding domain. The death of aging cells having high expression of NKG2D ligand is efficiently induced by means of bridging aging cells and immune cells, and thus the cell adapter molecules of the present application can be used for removing aging cells expressing the NKG2D ligand and treating aging-related diseases.

## Description

### Technical Field

The present invention relates to the field of biomedicine, and particularly relates to the use of NKG2D-CD3 and NKG2D-CD16 cell engager molecules in clearing senescent cells and treating related diseases.

### Background

Cellular senescence is a process in which cells enter a permanent state of cell cycle arrest in response to various stressors, occurring throughout the entire lifespan of an organism. While cellular senescence is related to individual aging, the two concepts are distinct. Even in elderly individuals, the number of senescent cells is quite low. When the immune system is strong enough to promptly clear senescent cells, the senescent cells can exist in the body briefly and exert beneficial functions such as promoting tissue regeneration, wound healing, and reducing cancer cell formation. However, when the organism undergoes the immune function decline or is continuously damaged, leading to an inability to timely clear senescent cells, the balance between the production and clearance of senescent cells is disrupted, resulting in their accumulation. Accumulated senescent cells not only directly impair the normal function of tissues and organs but also continuously secrete various inflammatory factors, causing tissue and organ damage and leading to various diseases such as fatty liver, liver fibrosis, pulmonary fibrosis, diabetes, osteoporosis, muscle atrophy, and atherosclerosis. Due to changes in modern lifestyles and poor dietary habits, the incidence of these diseases in younger individuals is gradually increasing, which has become a focus of societal concern. Preclinical data indicate that clearing accumulated senescent cells in human disease models such as mice, rats, and monkeys can delay, prevent, or alleviate over 40 related conditions. Additionally, clinical trials for treating diseases such as kidney fibrosis, diabetes, obesity, renal failure, age-related osteoporosis, and osteoarthritis by clearing senescent cells are also underway. Currently, methods for clearing senescent cells primarily focus on identifying small-molecule compounds that selectively clear senescent cells, such as dasatinib, quercetin, and ABT263. However, these compounds either have suboptimal efficacy in clearing senescent cells or exhibit significant toxic side effects. For example, ABT-263 can cause transient thrombocytopenia and neutropenia, and dasatinib may lead to severe pulmonary hypertension. Therefore, there is an urgent need in this field to develop a safer and more effective technical solution for clearing senescent cells. Bispecific antibodies have become a hotspot in drug development in recent years, with numerous biotech companies investing in their development, which has achieved breakthroughs in areas such as cancer, autoimmune diseases, and inflammatory diseases. Bispecific cell engagers are a type of bispecific antibody that selectively recruit immune effector cells (those for T cells are referred to as Bispecific T Cell Engagers, BiTEs; and those for NK cells are Bispecific NK Cell Engagers, BiKEs) to the vicinity of target cells, establishing immune synapses and activating immune effector cells to secrete effector molecules such as granzymes and perforins to clear target cells. Compared to traditional monoclonal antibodies, bispecific cell engagers offer higher sensitivity and specificity.

Thus, there is a need in this field to develop a technical solution for safely and effectively clearing senescent cells.

### Summary of the Invention

The purpose of the present invention is to provide a method for specifically and efficiently clearing senescent cells using NKG2D-CD3 and NKG2D-CD16 cell engager molecules.

In the first aspect of the present invention, it provides a use of a cell engager molecule targeting a NKG2D ligand and an immune cell for preparing a medicament used for:
(i) clearing senescent cells;
(ii) preventing and/or treating a disease associated with the accumulation of senescent cells;
(iii) delaying individual aging;

wherein the cell engager molecule comprises a first binding domain and a second binding domain,
wherein the first binding domain specifically binds to a NKG2D ligand, and the second binding domain specifically binds to a membrane protein on immune cell surface.

In another preferred embodiment, the first binding domain is an extracellular domain of NKG2D.

In another preferred embodiment, the first binding domain comprises a polypeptide as shown in SEQ ID NO: 1, or a polypeptide having more than 80% similarity to the sequence as shown in SEQ ID NO: 1 and capable of binding to a NKG2D ligand.

In another preferred embodiment, the second binding domain specifically binds to CD3 or CD16.

In another preferred embodiment, the cell engager molecule has a structure selected from the following formula (I) or (II) from N-terminus to C-terminus:

S-D₁-L₁-D₂-T (I);

or

S-D₂-L₁-D₁-T (II),

wherein,
each "-" is independently a linking peptide or peptide bond;
S is absent or a signal peptide sequence;
D₁ is the first binding domain;
Li is absent or a linker peptide;
D2 is the second binding domain;
T is absent or a marker protein.

In another preferred embodiment, the second binding domain has a structure selected from the group consisting of: single domain antibody (sdAb), single chain antibody (scFv), antigen-binding fragment (Fab fragment), ligand, or a polymer thereof, or a combination thereof.

In another preferred embodiment, the second binding domain is a CD3-binding domain which is capable of specifically binding to CD3 on the surface of T lymphocyte.

In another preferred embodiment, the CD3-binding domain has an amino acid sequence as shown in SEQ ID NO: 2, or has an amino acid sequence with at least 80%, 85%, 90%, 91%, 92%, 93%, 94%, 95%, 96%, 97%, 98%, or 99% sequence homology or sequence identity to the amino acid sequence as shown in SEQ ID NO: 2.

In another preferred embodiment, the second binding domain is a CD16-binding domain which is capable of specifically binding to CD16 on the surface of natural killer cell.

In another preferred embodiment, the CD16-binding domain has an amino acid sequence as shown in SEQ ID NO: 3 or 4, or has an amino acid sequence with at least 80%, 85%, 90%, 91%, 92%, 93%, 94%, 95%, 96%, 97%, 98%, or 99% sequence homology or sequence identity to the amino acid sequence as shown in SEQ ID NO: 3.

In another preferred embodiment, the CD16-binding domain is an Fc fragment of human IgG.

In another preferred embodiment, the Fc fragment of human IgG has an amino acid sequence as shown in SEQ ID NO: 5, or has an amino acid sequence with at least 80%, 85%, 90%, 91%, 92%, 93%, 94%, 95%, 96%, 97%, 98%, or 99% sequence homology or sequence identity to the amino acid sequence as shown in SEQ ID NO: 5.

In another preferred embodiment, the marker protein T is selected from: His tag, FLAG tag.

In another preferred embodiment, the linker peptide is a glycine-serine linker peptide.

In another preferred embodiment, the linker peptide is as represented by the formula (G₄S)n, where n is 1, 2, 3, 4, or 5.

In another preferred embodiment, the senescent cells are selected from the group consisting of: lung cells, fat cells, kidney cells, muscle cells, and a combination thereof.

In another preferred embodiment, the senescent cell is human embryonic lung cell HEL1.

In another preferred embodiment, the senescent cell is naturally occurring or artificially induced.

In another preferred embodiment, the artificial senescence induction method includes: senescence induction via DNA damage, senescence induction via P16 overexpression, senescence induction via telomere shortening, and a combination thereof.

In another preferred embodiment, the expression of NKG2D ligand is upregulated in the senescent cells.

In another preferred embodiment, the upregulation of NKG2D ligand expression means that the ratio of the expression level of NKG2D ligand in the senescent cells (F1) to that in normal cells (F0) (i.e., F1/F0) is ≥1.5, preferably ≥2, more preferably ≥2.5.

In another preferred embodiment, the medicament is used for preventing and/or treating a disease caused by the accumulation of senescent cells.

In another preferred embodiment, the NKG2D ligand includes (but is not limited to) MICA, MICB, ULBP1, ULBP2, ULBP3, ULBP4, ULBP5, ULBP6.

In another preferred embodiment, the age-related disease is selected from the group consisting of: heart failure, atherosclerosis, diabetes, myocardial hypertrophy, osteoporosis, tissue/organ fibrosis, Alzheimer's disease, Parkinson's syndrome, arthritis, and other degenerative diseases caused by cellular senescence, and a combination thereof.

In the second aspect of the present invention, it provides a pharmaceutical composition which comprises:
(a) a cell engager molecule targeting a NKG2D ligand and an immune cell;
(b) other drug for clearing senescent cells in addition to (a); and
(c) a pharmaceutically acceptable carrier, diluent, or excipient.

In another preferred embodiment, in component (b), the other anti-aging drugs include other drugs capable of specifically clearing senescent cells.

In another preferred embodiment, component (b) comprises a small molecule compound capable of specifically clearing senescent cells, preferably selected from the group consisting of: dasatinib, quercetin, ABT263, ABT737, piperlongumine, and a combination thereof.

In the third aspect of the present invention, it provides a pharmaceutical composition which comprises:
(a) a cell engager molecule targeting a NKG2D ligand and an immune cell;
(b) a drug capable of upregulating the expression of NKG2D ligands; and
(c) a pharmaceutically acceptable carrier, diluent, or excipient.

In another preferred embodiment, component (b) comprises a drug capable of upregulating the expression of NKG2D ligands on the cell surface, preferably selected from the group consisting of: etoposide, cisplatin, vincristine, paclitaxel, and a combination thereof.

In another preferred embodiment, the pharmaceutical composition is a liquid pharmaceutical composition.

In another preferred embodiment, the pharmaceutical composition is an injection.

It should be understood that, within the scope of the present invention, the technical features specifically described above and below (such as the Examples) can be combined with each other, thereby constituting a new or preferred technical solution which needs not be described one by one.

### DESCRIPTION OF DRAWINGS

The following drawings are used to illustrate specific embodiments of the present invention and are not intended to limit the scope of the present invention as defined by the claims.

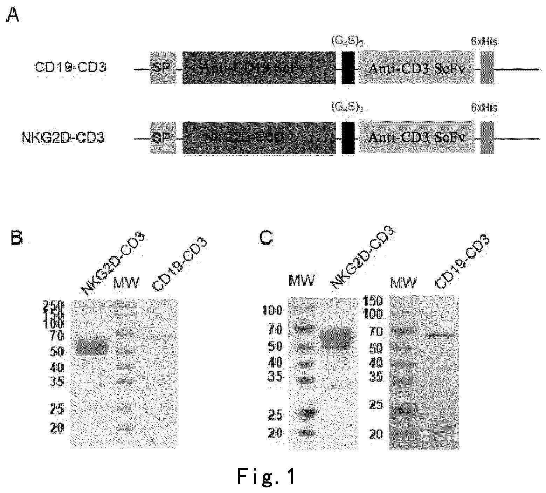

Figure 1 shows the preparation of NKG2D-CD3 protein. (A) Schematic diagram of the NKG2D-CD3 and CD19-CD3 vector structures. (B) After transfection of 293T cells with NKG2D-CD3 and CD19-CD3 vectors, the culture supernatant was collected, purified, and subjected to Coomassie brilliant blue staining. (C) The expression of NKG2D-CD3 and CD19-CD3 in the purified proteins was detected using His antibody.

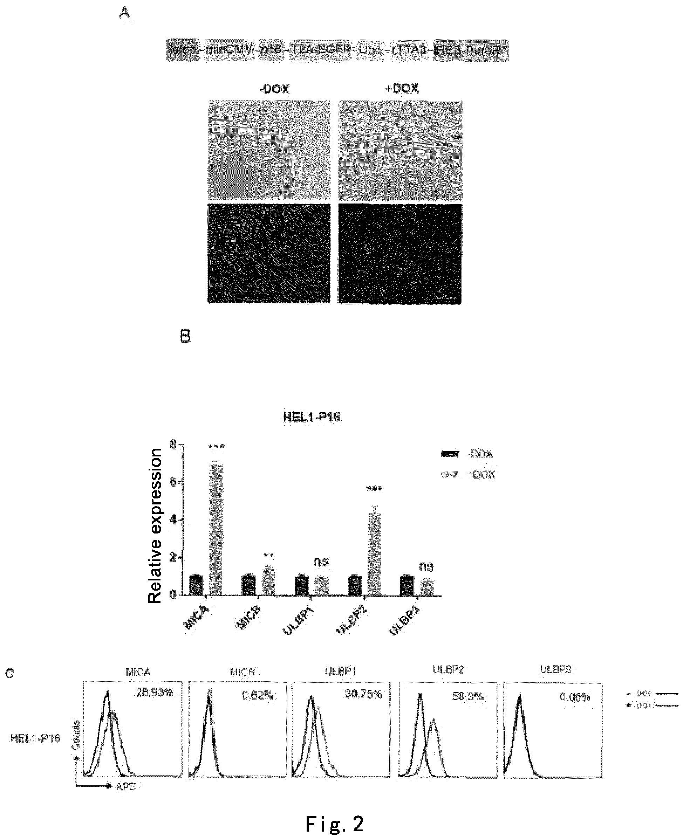

Figure 2 shows the upregulation of NKG2D ligand expression in senescent cells induced by P16 overexpression. (A) HEL1-P16 cells were induced to overexpress P16 using tetracycline (DOX) and subjected to β-gal staining. (B) HEL1-P16 cells were induced to overexpress P16 using DOX, and the expression of NKG2D ligand was detected by real-time PCR. (C) HEL1-P16 cells were induced to overexpress P16 using DOX, and the expression of NKG2D ligand was detected by flow cytometry.

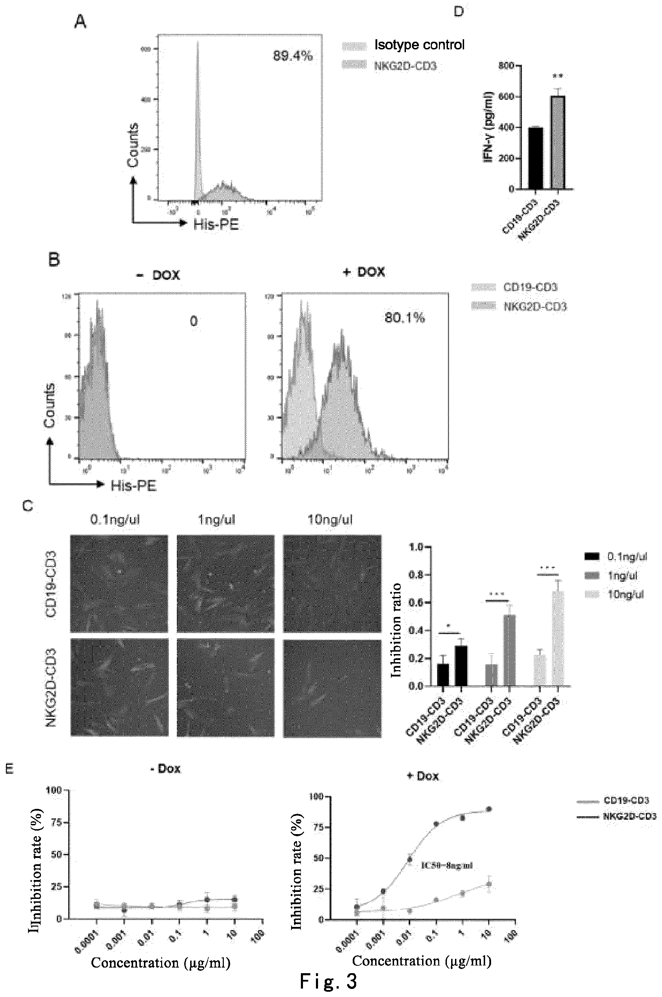

Figure 3 shows that NKG2D-CD3 promotes T cell-mediated killing of senescent cells induced by P16 overexpression. (A) After incubating NKG2D-CD3 with T cells, the binding rate was detected by flow cytometry. (B) After incubating NKG2D-CD3 with DOX-induced senescent HEL1-P16 cells, the binding rate was detected by flow cytometry. (C) After adding NKG2D-CD3 to a co-culture system of T cells and senescent cells for 8 hours, the mortality rate of senescent cells was detected. (D) After adding NKG2D-CD3 to a co-culture system of T cells and senescent cells for 8 hours, the culture supernatant was collected, and IFN-γ expression was detected by enzyme-linked immunosorbent assay (ELISA). (E) The detection of half-maximal inhibitory concentration (IC50) of NKG2D-CD3 against senescent cells induced by P16 overexpression.

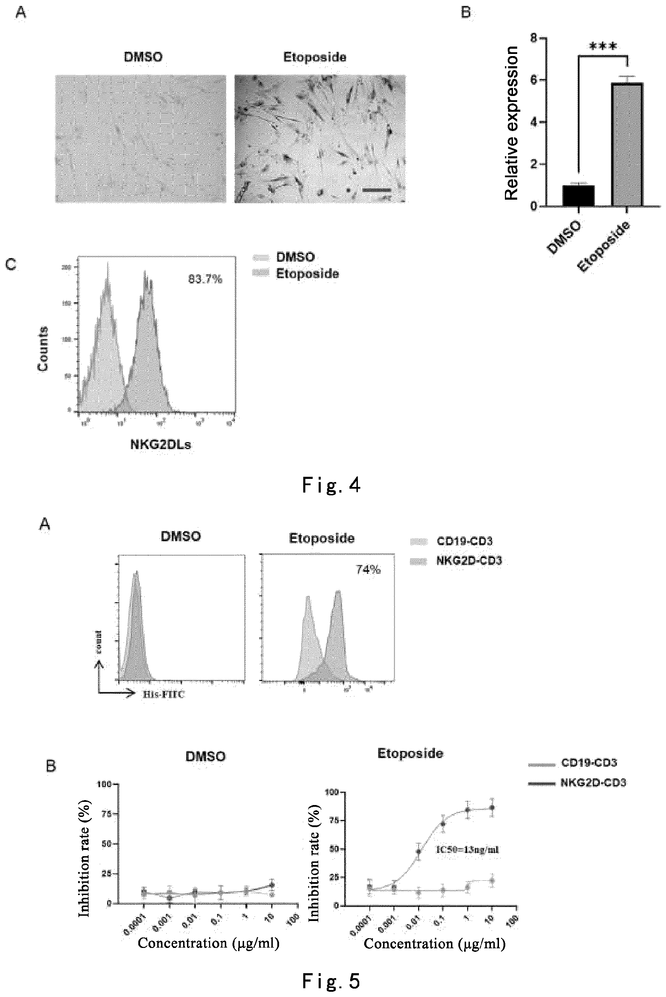

Figure 4 shows the upregulation of NKG2D ligand expression in senescent cells induced by DNA damage. (A) HEL1 cells were treated with etoposide to induce DNA damage and subjected to β-gal staining. (B) HEL1 cells were treated with etoposide to induce DNA damage, and the expression of NKG2D ligand MICA was detected by real-time PCR. (C) HEL1 cells were treated with etoposide to induce DNA damage, and the total NKG2D ligand expression was detected using NKG2D-FC fusion protein by flow cytometry.
Figure 5 shows that NKG2D-CD3 promotes T cell-mediated killing of senescent cells induced by DNA damage. (A) After incubating NKG2D-CD3 and CD19-CD3 antibodies with HEL1 cells treated with DMSO or Etoposide respectively, the binding rate was detected by flow cytometry. (B) After adding NKG2D-CD3 or CD19-CD3 antibodies to a co-culture system of T cells and HEL1 cells treated with DMSO or Etoposide, the half-maximal inhibitory concentration (IC50) against HEL1 cells was detected after 8 hours.

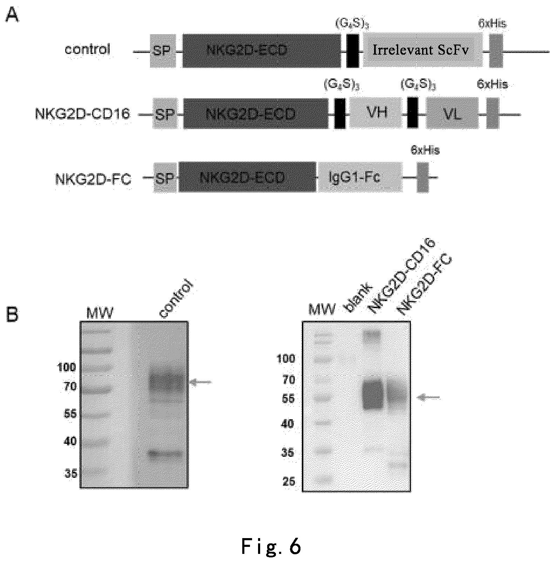

Figure 6 shows the preparation of NKG2D-CD16 and NKG2D-FC proteins. (A) Schematic diagram of the NKG2D-CD16, NKG2D-FC, and control vector structures. (B) After transfection of 293T cells with NKG2D-CD16, NKG2D-FC, and control vectors, the culture supernatant was collected and purified, and the expression of NKG2D-NKp46 and control proteins in the purified proteins was detected using His antibody.

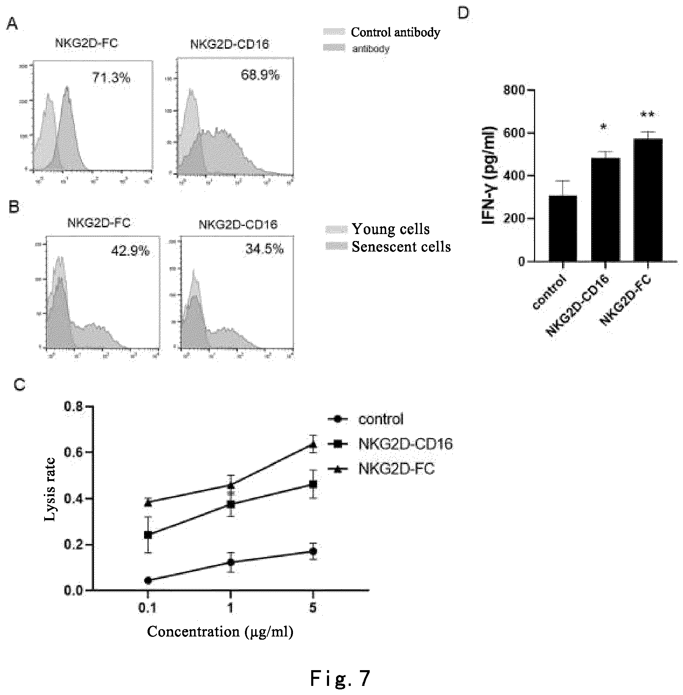

Figure 7 shows that NKG2D-CD16 and NKG2D-FC proteins promote NK cell-mediated killing of senescent cells. (A) After incubating NKG2D-CD16, NKG2D-FC, and control vectors with NK cells respectively, the binding rate was detected by flow cytometry. (B) After incubating NKG2D-CD16 and NKG2D-FC with young cells and etoposide-induced senescent cells, the binding rate was detected by flow cytometry. (C) After adding NKG2D-CD16, NKG2D-FC, and control vectors to a co-culture system of NK cells and senescent cells for 8 hours, the mortality rate of senescent cells was detected. (D) After adding NKG2D-CD16, NKG2D-FC, and control vectors to a co-culture system of NK cells and senescent cells for 8 hours, the culture supernatant was collected, and IFN-γ expression was detected by ELISA.

### EMBODIMENTS

Through extensive and intensive research and a large amount of screening, the present inventors have developed, for the first time, a method to specifically and efficiently clear senescent cells and treat age-related diseases in subjects, using a bispecific cell engager technology.

The bispecific cell engager molecule provided by the present invention consists of three parts: the NKG2D extracellular domain targeting NKG2D ligands on the surface of senescent cells, a linker segment, and a CD3 or CD16 domain binding to immune cells. The engager molecule connects senescent cells through the NKG2D extracellular domain while bridging immune cells, thereby directing effector immune cells to the surface of senescent cells to achieve the goal of clearing senescent cells.

### Terms

To make it easier to understand the present invention, certain technical and scientific terms are specifically defined below. Unless otherwise clearly defined herein, all other technical and scientific terms used herein have meanings commonly understood by those skilled in the art in the field to which the present invention belongs. Before describing the present invention, it should be understood that the present invention is not limited to the specific methods and experimental conditions described, as such methods and conditions can be changed. It should also be understood that the terms used herein are only intended to describe the specific embodiments and are not intended to be restrictive. The scope of the present invention will be limited only by the accompanying claims.

Unless otherwise defined, all technical and scientific terms used herein have the same meaning as would normally be understood by those of ordinary skill in the art to which the invention belongs. As used herein, term "about" means that the value may change by no more than 1% from the enumerated value when used in reference to a specific enumerated value. For example, as used herein, the expression "about 100" includes all values between 99 and 101 and (e. g., 99.1, 99.2, 99.3, 99.4, etc.).

The three-letter and single-letter amino acid codes used in the present invention are as described in J. Biol. Chem., 243, p3558 (1968).

As used herein, the term "treatment" refers to the administration of an internal or external therapeutic agent, including the bispecific cell engager and composition thereof of the present invention, to a patient exhibiting one or more disease symptoms, wherein the therapeutic agent is known to have a therapeutic effect on these symptoms. Typically, the therapeutic agent is administered in an amount sufficient to alleviate one or more disease symptoms (a therapeutically effective amount).

As used herein, the term "optional" or "optionally" means that the event or situation described thereafter may occur, but not necessarily. For example, "optionally comprising 1-3 antibody heavy chain variable regions" means that the antibody heavy chain variable region of a specific sequence may be comprised but does not have to be comprised, and the number of it can be 1, 2 or 3.

The "sequence identity" described in the present invention refers to the degree of identity between two nucleic acid sequences or two amino acid sequences with appropriate mutations such as substitutions, insertions, or deletions when optimally aligned and compared. The sequence identity between a sequence described in the present invention and a sequence having identity thereto may be at least 85%, 90%, or 95%, preferably at least 95%. Non-limiting examples include 85%, 86%, 87%, 88%, 89%, 90%, 91%, 92%, 93%, 94%, 95%, 96%, 97%, 98%, 99%, and 100%.

### First Binding Domain

The first binding domain of the cell engager for use in the present invention specifically binds to a NKG2D ligand (NKG2DL).

The NKG2D-NKG2DL signaling pathway is an important mechanism for the immune surveillance function of the body's immune system. Bispecific cell engager molecules NKG2D-CD3 and NKG2D-CD16, constructed based on the extracellular domain of NKG2D, have also gradually gained attention in the field of anti-tumor therapy. Studies have shown that NKG2D-CD3 and NKG2D-CD16 can activate T cells and NK cells, respectively, and efficiently kill tumor cells with high expression of NKG2D ligands *in vitro,* such as acute myeloid leukemia, lymphoma, soft tissue sarcoma, and melanoma. NKG2D-CD3 can inhibit tumor growth and prolong the survival of model mice *in vivo* by targeting tumor cells and immunosuppressive cells. The expression of NKG2D ligands is also significantly upregulated in senescent cells.

The first binding domain of the cell engager of the present invention is preferably derived from the extracellular domain of NKG2D. In a preferred embodiment, the NKG2D extracellular domain comprises a polypeptide as shown in SEQ ID NO: 1, or a polypeptide having more than 80% similarity (preferably more than 85%, more preferably more than 90%) to the sequence as shown in SEQ ID NO: 1 and capable of binding to NKG2D ligands.

### Second Binding Domain

The second binding domain of the cell engager for use in the present invention specifically binds to a membrane protein on immune cell surface. The membrane protein on immune cell surface is preferably CD3 or CD16.

The second binding domain comprised in the cell engager molecule of the present invention may be an antibody, an antigen-binding fragment of an antibody, or any other polypeptide capable of specifically binding to CD3 or CD16.

As used herein, the term "antibody" may be an immunologically active antibody fragment, such as a Fab or (Fab')2 fragment; an antibody heavy chain; or an antibody light chain. In a preferred embodiment, the antibody used in the present invention is in the form of a single-chain antibody (scFv), which contains the heavy chain variable region and the light chain variable region of an antibody but lacks the constant region, and is the smallest antibody fragment with complete antigen-binding sites. Generally, the Fv antibody also comprises a polypeptide linker between the VH and VL domains and is capable of forming the structure required for antigen binding. In another preferred embodiment, the antibody used in the present invention is in the form of a single domain antibody (VhH), which consists of only one heavy chain variable region of a cloned antibody and is the smallest antigen-binding fragment with full function.

The terms "specifically binds", "selectively binds", "selectively binding", and "specifically binding" refer to the binding of an antibody to an epitope on a predetermined antigen. Typically, the antibody binds with an affinity (KD) of less than about 10⁻⁷ M, for example, less than about 10⁻⁸ M, 10⁻⁹ M, or 10⁻¹⁰ M, or even lower.

In a preferred embodiment of the present invention, the second binding domain is a CD3-binding domain which is capable of specifically binding to CD3 on the surface of T lymphocyte. The CD3-binding domain has an amino acid sequence as shown in SEQ ID NO: 2, or has an amino acid sequence with at least 80%, 85%, 90%, 91%, 92%, 93%, 94%, 95%, 96%, 97%, 98%, or 99% sequence homology or sequence identity to the amino acid sequence as shown in SEQ ID NO: 2.

In a preferred embodiment of the present invention, the second binding domain is a CD 16-binding domain which is capable of specifically binding to CD16 on the surface of natural killer cell. In one embodiment, the CD16-binding domain is an anti-CD16 scFv or single-domain antibody, which has the amino acid sequence as shown in SEQ ID NO: 3 or SEQ ID NO: 4 or an amino acid sequence with at least 80%, 85%, 90%, 91%, 92%, 93%, 94%, 95%, 96%, 97%, 98%, or 99% sequence homology or sequence identity to the amino acid sequence as shown in SEQ ID NO: 3 or SEQ ID NO: 4. In another embodiment, the CD16-binding domain is an Fc fragment of human IgG, which has the amino acid sequence as shown in SEQ ID NO: 5 or an amino acid sequence with at least 80%, 85%, 90%, 91%, 92%, 93%, 94%, 95%, 96%, 97%, 98%, or 99% sequence homology or sequence identity to the amino acid sequence as shown in SEQ ID NO: 5.

### Bispecific Cell Engager Molecule

As used herein, the terms "bispecific cell engager molecule", "bispecific cell engager", "cell engager", "BiTE", and "bispecific antibody" are used interchangeably and all refer to the cell engager molecule provided in the first aspect of the present invention, which is capable of simultaneously binding to a NKG2D ligand and an immune cell-expressed protein.

Bispecific cell engager molecules are formed by connecting two proteins or polypeptide sequences (most commonly antibodies) that bind to different target proteins. In a preferred embodiment, the function of the BiTE of the present invention is determined by the extracellular domain of the NKG2D receptor and the CD3 or CD16 protein-binding segment. The antibody of the present invention can simultaneously bind to a NKG2D ligand and the membrane protein CD3 or CD16 on immune cell surfaces, connect senescent cells through the ligand segment and connect immune cells through the anti-immune cell surface membrane protein segment, thereby effectively bridging effector immune cells with senescent cells in order to more efficiently clear senescent cells.

As used herein, the term "bispecific" refers to a molecule containing at least two binding domains with different binding specificities. Each binding domain is capable of specifically binding to a target molecule. In some embodiments, the bispecific cell engager is a polymeric molecule with two or more peptides. In some embodiments, the binding domain includes the antigen-binding domain of an antibody, or a variable region, or CDR. In some embodiments, the binding domain includes a ligand that specifically binds to the target protein, or a fragment thereof.

The at least two targeting domains of the cell engager molecule of the present invention are optionally connected by a linker peptide. A preferred linker peptide sequence is (G₄S)₃, but is not limited thereto.

In a preferred embodiment of the present invention, the bispecific cell engager molecule is a single-chain polypeptide, which comprises, the extracellular domain segment of NKG2D as the first binding domain, a linker segment, and an anti-CD3 or anti-CD16 single-chain antibody as the second binding domain, wherein the anti-CD3 or anti-CD16 single-chain antibody is a conventional single-chain antibody in the art which comprises a heavy chain variable region and a light chain variable region.

At the same time, it should be understood by those skilled in the art that although the first binding domain of the BiTE of the present invention is preferably the extracellular domain segment of NKG2D, the first binding domain may also be selected to be an antibody that specifically binds to NKG2D or an antigen-binding fragment thereof, as long as it can achieve the cell engager effect of the present invention.

The antibody of the present invention may be a chimeric antibody, a humanized antibody, or a CDR-grafted and/or modified antibody targeting a NKG2D ligand and CD3 (e.g., a human NKG2D ligand and CD3).

The antibody of the present invention may be a chimeric antibody, a humanized antibody, or a CDR-grafted and/or modified antibody targeting a NKG2D ligands and CD16 (e.g., a human NKG2D ligand and CD16).

In the present invention, the BiTE of the present invention further includes conservative variants thereof, which refer to polypeptides that comprises at most 10, preferably at most 8, more preferably at most 5, most preferably at most 3 amino acids replaced by amino acids with the same or similar properties compared with the amino acid sequence of the antibody of the present invention. These conservatively variant polypeptides are preferably produced by amino acid substitution according to Table 1.

**Table 1**

| Initial residue | Representative substitution | Preferred substitution |
|---|---|---|
| Ala (A) | Val; Leu; Ile | Val |
| Arg (R) | Lys; Gln; Asn | Lys |
| Asn (N) | Gln; His; Lys; Arg | Gln |
| Asp (D) | Glu | Glu |
| Cys (C) | Ser | Ser |
| Gln (Q) | Asn | Asn |
| **Glu** (E) | Asp | Asp |
| Gly (G) | Pro; Ala | Ala |
| His (H) | Asn; Gln; Lys; Arg | Arg |
| Ile (I) | Leu; Val; Met; Ala; Phe | Leu |
| Leu (L) | Ile; Val; Met; Ala; Phe | Ile |
| Lys (K) | Arg; Gln; Asn | Arg |
| Met (M) | Leu; Phe; Ile | Leu |
| Phe (F) | Leu; Val; Ile; Ala; Tyr | Leu |
| Pro (P) | Ala | Ala |
| Ser (S) | Thr | Thr |
| Thr (T) | Ser | Ser |
| Trp (W) | Tyr; Phe | Tyr |
| Tyr (Y) | Trp; Phe; Thr; Ser | Phe |
| Val (V) | Ile; Leu; Met; Phe; Ala | Leu |

Moreover, the amino acid sequences also include sequences formed by the addition, deletion, modification, and/or substitution of at least one amino acid, preferably sequences with homology or sequence identity of at least 80%, more preferably at least 85%, even more preferably at least 90%, and most preferably at least 95%.

Methods for determining sequence homology or identity well-known to those skilled in the art include, but are not limited to: Computational Molecular Biology, edited by Lesk, A.M., Oxford University Press, New York, 1988; Biocomputing: Informatics and Genome Projects, edited by Smith, D.W., Academic Press, New York, 1993; Computer Analysis of Sequence Data, Part I, edited by Griffin, A.M. and Griffin, H.G., Humana Press, New Jersey, 1994; Sequence Analysis in Molecular Biology, von Heinje, G., Academic Press, 1987; and Sequence Analysis Primer, edited by Gribskov, M. and Devereux, J., M Stockton Press, New York, 1991 and Carillo, H. and Lipman, D., SIAM J. Applied Math.48:1073(1988). The preferred method for determining identity shall maximize the alignment between the sequences being tested. Methods for determining identity are compiled into publicly available computer programs. Preferred computer programs for determining the identity between two sequences include, but are not limited to: the GCG package (Devereux, J. *et al.,* 1984), BLASTP, BLASTN, and FASTA (Altschul, S.F. *et al.,* 1990). The BLASTX program is publicly available from NCBI and other sources (BLAST Manual, Altschul, S. *et al.,* NCBI NLM NIH Bethesda, Md. 20894; Altschul, S. *et al.,* 1990). The well-known Smith-Waterman algorithm can also be used to determine identity.

In the aforementioned context of the present invention, the number of added, deleted, modified, and/or substituted amino acids is preferably not more than 40% of the total number of amino acids in the initial amino acid sequence, more preferably not more than 35%, more preferably 1-33%, more preferably 5-30%, more preferably 10-25% and more preferably 15-20%.

In the aforementioned context of the present invention, more preferably, the number of amino acids added, deleted, modified, and/or substituted may be 1-7, more preferably 1-5, even more preferably 1-3, and most preferably 1-2.

### Pharmaceutical composition

The present invention also provides a composition. In a preferred embodiment, the composition is a pharmaceutical composition comprising the aforementioned cell engager or an active fragment thereof, or a fusion protein thereof, or an ADC thereof, or a corresponding immune cell, and a pharmaceutically acceptable carrier.

In one embodiment of the present invention, it provides a pharmaceutical composition which comprises:
(a) a cell engager molecule binding a NKG2D and a membrane protein on immune cell surface;
(b) other drug for clearing senescent cells in addition to (a); and
(c) a pharmaceutically acceptable carrier, diluent, or excipient.

In a preferred embodiment, component (b) comprises a small molecule compound capable of specifically clearing senescent cells, preferably selected from the group consisting of: dasatinib, quercetin, ABT263, ABT737, piperlongumine, and a combination thereof.

In another embodiment of the present invention, it provides a pharmaceutical composition which comprises:
(a) a cell engager molecule binding a NKG2D and a membrane protein on immune cell surface;
(b) a drug capable of upregulating the expression of NKG2D ligands; and
(c) a pharmaceutically acceptable carrier, diluent, or excipient.

In a preferred embodiment, component (b) comprises a drug capable of upregulating the expression of NKG2D ligands on the cell surface, preferably selected from the group consisting of: etoposide, cisplatin, vincristine, paclitaxel, and a combination thereof.

Typically, these substances can be formulated in non-toxic, inert, and pharmaceutically acceptable aqueous carrier media, wherein the pH is generally about 5-8, preferably about 6-8, although the pH value may vary depending on the nature of the substance being formulated and the condition to be treated.

The formulated pharmaceutical composition can be administered via conventional routes, including but not limited to: intramuscular, intraperitoneal, intravenous, or topical administration. Typically, the route of administration for the pharmaceutical composition of the present invention is preferably injection or oral administration. The injection administration preferably includes intravenous, arterial, intramuscular, intraperitoneal, intradermal, or subcutaneous injection routes. The pharmaceutical composition is in various conventional dosage forms in the art, preferably in solid, semi-solid, or liquid forms, and can be aqueous solutions, non-aqueous solutions, or suspensions, more preferably tablets, capsules, granules, injections, or infusions.

The pharmaceutical composition according to the present invention comprises a safe and effective amount (e.g. 0.001-99 wt %, preferably 0.01-90 wt %, preferably 0.1-80 wt %) of the monoclonal antibody according to the present invention (or a conjugate thereof) and a pharmaceutically acceptable carrier or excipient. Such carriers include (but are not limited to): saline, buffers, glucose, water, glycerol, ethanol, and a combination thereof. Pharmaceutical preparations should correspond to the administration modes. The pharmaceutical composition according to the present invention can be prepared in the form of an injection, for example, by a conventional method using physiological saline or an aqueous solution containing glucose and other adjuvants. A pharmaceutical composition, for example, an injection and a solution, should be prepared under aseptic conditions. The administration amount of an active ingredient is a therapeutically effective amount, for example, about 1 µg per kilogram of body weight to about 5 mg per kilogram of body weight daily. In addition, the cell engager according to the present invention may also be used in combination with an additional therapeutic agent.

In the present invention, preferably, the pharmaceutical composition of the present invention further comprises one or more pharmaceutically acceptable carriers. The pharmaceutically acceptable carrier is a conventional pharmaceutically acceptable carrier in the art, and can be any suitable physiologically or pharmaceutically acceptable excipient. The excipient is a conventional excipient in the art, preferably including pharmaceutically acceptable excipients, fillers, or diluents. More preferably, the pharmaceutical composition comprises 0.01% to 99.99% of the aforementioned protein and 0.01% to 99.99% of the pharmaceutically acceptable carrier, wherein the percentages are mass percentages in the pharmaceutical composition.

In the present invention, preferably, the dosage of the pharmaceutical composition is an effective amount, wherein the effective amount is an amount capable of alleviating or delaying the progression of a disease, degenerative condition, or injury condition. The effective amount can be determined on an individual basis and will be partially based on considerations of the condition to be treated and the desired outcome. Those skilled in the art can determine the effective amount by using the aforementioned factors on an individual basis and using no more than routine experimentation.

When a pharmaceutical composition is used, a safe and effective amount of Immune conjugate is administered to a mammal, wherein the safe and effective amount is generally at least about 10 µg per kilogram of body weight, and in most cases, no more than about 50 mg per kilogram of body weight, preferably, the amount is from about 10 µg per kilogram of body weight to about 20 mg per kilogram of body weight. Of course, a specific amount should also depend on the factors such as administration route and physical conditions of a patient, which fall into the skills of skilled physicians.

### Therapeutic Application

The present invention provides the use of the NKG2D ligand-targeting cell engager molecule and the pharmaceutical composition of the present invention for the prevention and/or treatment of age-related diseases.

Furthermore, the present invention also provides the use of the NKG2D-targeting cell engager molecule and the pharmaceutical composition of the present invention for the preparation of a medicament for use in (i) clearing senescent cells; (ii) delaying individual aging; and/or (iii) preventing and/or treating age-related diseases.

In a preferred embodiment, the NKG2D ligand in the senescent cells is upregulated by more than 1.5-fold compared to that in normal cells.

Wherein the age-related diseases include: muscle atrophy, fatty liver, heart failure, atherosclerosis, diabetes, myocardial hypertrophy, osteoporosis, tissue/organ fibrosis, Alzheimer's disease, Parkinson's syndrome, arthritis, and other organ degenerative diseases caused by cellular senescence, or a combination thereof.

The universal cell engager molecule of the present invention can also be used as a type of vaccine for *ex vivo* immunization and/or *in vivo* therapy of mammals. Preferably, the mammal is a human.

In addition to the use of cell-based vaccines for *ex vivo* immunization for cells, the present invention also provides compositions and methods for *in vivo* enhancing immune responses against target antigens in patients.

Pharmaceutical compositions of the present invention may be administered in a manner appropriate to the disease to be treated (or prevented). The quantity and frequency of administration will be determined by such factors as the character of the condition of the patient, and the type and severity of the patient's disease, although appropriate dosages may be determined by clinical trials.

When referring to "an immunologically effective amount", "an anti-aging effective amount", "a senescence-related disease-inhibiting effective amount", or "a therapeutic amount", the precise amount of the composition of the present invention to be administered can be determined by a physician, taking into account the individual differences in the patient's (subject's) age, weight, size of senescent tissue, degree of senescence, and condition.

### Main advantages of the present invention

The bispecific cell engager molecule constructed in the present invention simultaneously targets NKG2D ligands and immune cells. It can be directly infused into the body or carried by cells in the body (such as NK cells, T lymphocytes, CAR-T cells, etc.) and continuously expressed in the body, thereby enabling the bispecific cell engager molecule to exert its cytotoxic effects *in vivo.* The main advantages include:
1) High targeting ability: The bispecific cell engager molecule targeting NKG2D ligand-positive senescent cells can effectively bridge senescent cells and immune cells, with stable binding and strong cytotoxicity.
2) High safety: NKG2D ligands are important targets for natural immune cells to clear senescent cells and tumor cells, and their expression on the surface of normal cells is strictly regulated. The NKG2D-NKG2DL signaling pathway has undergone long-term natural selection, ensuring high safety. Furthermore, a large number of clinical trials targeting NKG2D ligands are currently underway, and no serious treatment-related side effects have been observed.

The invention is further illustrated below in conjunction with specific embodiments. It should be understood that the examples are not intended to limit the scope of the invention. The experimental methods in the following examples which do not specify the specific conditions are usually in accordance with conventional conditions, such as conditions described in Sambrook et al., Molecular Cloning: Laboratory Manual (New York: Cold Spring Harbor Laboratory Press, 1989), or in accordance with the conditions recommended by the manufacturer. Unless otherwise stated, percentages and parts are by weight.

The sequences involved in the Examples of the present invention are as follows:
SEQ ID NO: 1 NKG2D extracellular domain 
FLNSLFNQEVQIPLTESYCGPCPKNWICYKNNCYQFFDESKNWYESQASC
MSQNASLLKVYSKEDQDLLKLVKSYHWMGLVHIPTNGSWQWEDGSILSPNLL
TIIEMQKGDCALYASSFKGYIENCSTPNTYICMQRTV
SEQ ID NO: 2 CD3 binding domain (derived from the CD3 monoclonal antibody OKT3) 
DIKLQQSGAELARPGASVKMSCKTSGYTFTRYTMHWVKQRPGQGLEWIG
YINPSRGYTNYNQKFKDKATLTTDKSSSTAYMQLSSLTSEDSAVYYCARYYDD
HYCLDYWGQGTTLTVSSVEGGSGGSGGSGGSGGVDDIQLTQSPAIMSASPGEK
VTMTCRASSSVSYMNWYQQKSGTSPKRWIYDTSKVASGVPYRFSGSGSGTSYS
LTISSMEAEDAATYYCQQWSSNPLTFGAGTKLELK
SEQ ID NO: 3 CD16 binding domain scFv fragment (cited from patent US 11167029B2) 
QVQLVQSGAEVKKPGESLKVSCKASGYTFTSYYMHWVRQAPGQGLEWM
GIINPSGGSTSYAQKFQGRVTMTRDTSTSTVYMELSSLRSEDTAVYYCARGSAY
YYDFADYWGQGTLVTVSSGGGGSGGGGSGGGGSSYVLTQPSSVSVAPGQTATI
SCGGHNIGSKNVHWYQQRPGQSPVLVIYQDNKRPSGIPERFSGSNSGNTATLTIS
GTQAMDEADYYCQVWDNYSVLFGGGTKLTVL
SEQ ID NO: 4 CD16 binding domain VhH fragment (cited from GenBank ABS29544.1) 
EVQLVESGGGFVQAGESLTLSCTSSTLTFTPYRMAWYRQAPGKQRDLVAD
ISSGDGRTTNYADFAKGRFTISRDNIKNTVFLRMTNLKPEDTAVYYCNTFVSFV
GIARSWGQGTQVTVSS
SEQ ID NO: 5 Fc fragment of IgG antibody (derived from mogamulizumab)

PKSCDKTHTCPPCPAPELVGGPSVFLLPPKPKDTLMISRTPEVTCVVVDVSH
EDPEVKFNWYVDGVEVHNAKTKPPEEQYNSTLRVVSVLTVLHQDWLNGKEYK
CKVSNKALPAPIEKTISKAKGQPREPQVYTLPPSRDELTKNQVSLTCLVKGFYPS
DIAVEWESNGQPENNYKTTPLVLDSDGSFFLYSKLTVDKSRWQQGNVFSCSVM

HEALHNHYTQKSLSLSPGK

### Example 1 Preparation of NKG2D-CD3 Protein

### 1.1 Vector Construction

The nucleotide sequence of the target gene with the structure shown in Fig. 1A was synthesized and cloned into the lentiviral vector pCDH-CMV-MCS-EF1-Puro (refer to Myeloid Leukemia. Mol Ther, 2016. 24(9): p. 1615-26.) through EcoR I and Swa I restriction sites. After verifying the correctness of the cloned vector through restriction enzyme digestion and sequencing, it was transformed into competent *E. coli* (Stbl3) and expanded for culture. The plasmid was then extracted using the QIAGEN endotoxin-free midi extracting kit and identified by Hind III restriction enzyme digestion.

### 1.2 Virus Package

HEK-293 T cells were cultured in a 15 cm culture dish for virus package. Transfection was carried out when the confluence of HEK-293T cells was about 90%. A plasmid mixture dissolved in 2ml OPTIMEM was prepared (core plasmid 20ug, pCMVΔR8.9 10ug, PMD2.G 4ug); and 2ml OPTIMEM and 68ul of lipo 8000 were combined in another tube. After set at room temperature for 5 minutes, the plasmid complex was added to the liposome complex and set at room temperature for 20 minutes. The mixture was then added dropwise to the HEK-293T cells and incubated at 37°C for 6 hours before removing the medium. Pre-warmed complete media were added. Viral supernatants were collected 48 hours and 72 hours after transfection, centrifuged at 3000 rpm for 20 minutes at 4 °C, filtered through a 0.45um filter membrane, and then concentrated by centrifugation at 25000rpm for 2.5 hours at 4 °C. The concentrated virus was dissolved overnight in 30 ul of virus dissolving solution, and the virus titer was detected by QPCR. The results show that the viral titer met the requirements.

### 1.3 Protein Preparation and Purification

The CHO cells were infected with the aforementioned virus, and after 24 hours, 1 µg/mL puromycin was added for 48 hours of selection. After selection, the CHO cells were further cultured for 7 days. The culture supernatant was collected and filtered through a 0.22 µm membrane. The His-tagged antibody was obtained from the expression supernatant using an affinity chromatography column. The equilibration buffer was 20 mM sodium phosphate buffer containing 0.5 M NaCl (900 mL, pH 7.4). The elution buffer was 20 mM sodium phosphate buffer containing 0.5 M imidazole and 0.5 M NaCl (900 mL, pH 7.4). The NKG2D-CD3 bispecific antibody was obtained by passing through a cation exchange column and was finally concentrated by buffer exchange with PBS. The SDS-PAGE electrophoresis of the purified NKG2D-CD3 protein is shown in Fig. 1B. The Western Blot results shown in Fig. 1C indicate that the molecular weight matches the theoretical value.

### Example 2 Upregulation of NKG2D Ligand Expression in Senescent Cells

### 2.1 Construction of a Cell Senescence Model Overexpressing p16 Protein with Tet-on System

(1) 3 × 10⁵ cells were seeded in 10 cm dishes, and on the next day, the cell confluence reached approximately 20% after attachment;
(2) After cell attachment, the cells were infected with lentivirus overexpressing p16 protein using the Tet-on system at a multiplicity of infection (MOI) of 50-100. Polybrene (stock concentration: 8 mg/mL) was added at a ratio of 1:1000 to enhance infection efficiency;
(3) After 24 hours, the cells were subjected to a second infection with the same amount of virus;
(4) Four days after infection, the cells were selected with puromycin at a final concentration of 3 µg/mL;
(5) The constructed p16-overexpressing cells were passaged into well plates or dishes. After 24 hours of attachment, 1 µg/mL dox was added to induce p16 protein expression;
(6) After 8 days of induction, the cells were stained using the SA-β-gal staining kit (CS0030, Sigma) for detecting senescence. As shown in Fig. 2A, over 90% of the cells were positive, indicating that the cells had become senescent.

### 2.2 Construction of a Cell Senescence Model Induced by DNA-Damaging Drug

(1) Cells were seeded in a 10 cm dish to achieve approximately 50% confluence after attachment;
(2) After 24 hours, Etoposide (sigma, E1383) was added to a final concentration of 50 µM;
(3) After 36 hours, the medium was replaced with fresh medium;
(4) The cells were continued to be cultured, with the medium replaced every three days. After 8 days, the cells exhibited a senescent phenotype. The cell senescence was detected using the SA-β-gal staining kit (CS0030, Sigma). As shown in Fig. 4A, over 90% of the cells were positive, indicating that the cells had become senescent.

### 2.3 Detection of NKG2D Ligand Expression at the Transcriptional Level

(1) After preparing senescent cells as described above, 1~2 mL of Trizol was added to the 10 cm dishes based on cell density, and the dishes were placed on ice for 5 minutes. The cells were then mixed by pipetting;
(2) 1 mL of the lysate from each dish was transferred to a 1.5 mL EP tube, and 200 µL of chloroform was added. The mixture was shaken with force for 15 seconds and then placed at room temperature for 5 minutes, followed by centrifugation (4°C, 12,000 g, 15 minutes);
(3) 450 µL of isopropanol was added to a new EP tube;
(4) After centrifugation, the upper colorless layer was carefully transferred to the EP tube containing isopropanol, mixed, and incubated at room temperature for 10 minutes, followed by centrifugation (4°C, 12,000 g, 10 minutes);
(5) The supernatant was discarded, and the RNA was washed with 1 mL of 75% ethanol prepared with RNase-free water, followed by centrifugation (4°C, 7,500 g, 5 minutes);
(6) The supernatant was carefully discarded, and the tube was inverted to air dry for 5 minutes. Any remaining liquid on the tube walls was removed using a pipette tip;
(7) The RNA was dissolved in 30 µL of RNase-free water, immediately placed on ice, and its concentration was measured;
(8) Using the extracted RNA as a template, 2 µg of RNA was reverse transcribed into cDNA using the Thermo Scientific RevertAidTM First Strand cDNA Synthesis Kit. The reaction system was as follows:

| Component | Amount |
|---|---|
| RNA Template | 2µg |
| Random Primer | 1 µL |
| RNase-free H₂O | ~ 12µL |
| Total Volume | 12µL |

(9) The reaction mixture was added to a PCR tube according to the above system and placed in a PCR machine at 65 °C for 5 minutes, then immediately placed on ice. The following components were then added to the tube:

| Component | Amount |
|---|---|
| 5x reaction buffer | 4µL |
| RiboLock RNase Inhibitor (20 U/µL) | 1µL |
| 10mM dNTP Mix | 2µL |
| RevertAid Reverse Transcriptase (200 U/µL) | 1µL |
| Total volume | 20µL |

The mixture was gently mixed and short spun, then placed in the PCR machine for the following reaction: 25°C for 5 minutes; 42°C for 1 hour; 70°C for 5 minutes;
(10) The expression of NKG2D ligands was detected by real-time quantitative PCR using the Thermo powerup^{™} SYBR Green Master Mix (A25742) kit according to the manufacturer's instructions. The program was as follows: 50°C for 2 minutes; 95°C for 2 minutes; 95°C for 15 seconds (40 cycles); 60°C for 1 minute (40 cycles); 12°C, forever;
(11) The data were exported in Excel format, and the relative expression levels of NKG2D ligands were calculated. As shown in Figs. 2B and 4B, the expression of NKG2D ligands was significantly upregulated.

### 2.4 Detection of NKG2D Ligand Expression Level on Membrane

(1) HEIP cells were collected by trypsin digestion, washed three times with 1 x PBS, and resuspended in 200 µL of 1x PBS (containing 2% FBS) to adjust the cell concentration to 1x10⁶ cells/mL.
(2) The resuspended cells was added with NKG2D ligand antibody, mixed well, and incubated on ice for 120 minutes. Cells were subjected to vortex every 10 minutes during incubation. The cells were then centrifuged at 500 g for 5 minutes, and the supernatant was discarded;
(3) The cells were resuspended in 1 mL of 1x PBS (containing 2% FBS) and centrifuged at 500 g for 5 minutes;
(4) The step was repeated;
(5) The expression of NKG2D ligands was detected by flow cytometry. The results are shown in Figs. 2C and 4C.

### Example 3 NKG2D-CD3 Protein Promotes the T Cell-Mediated Killing of Senescent Cells

### 3.1 Isolation of Human Peripheral Blood T Lymphocytes

(1) Human peripheral blood was transferred to a 50 mL centrifuge tube, and RosetteSep^{™} Cocktail was added to the blood (50 µL/mL blood);
(2) After thorough mixing, the sample was incubated at room temperature for 20 minutes;
(3) A diluent was prepared by mixing 1640 medium with 1x PBS at a volume ratio of 1:2;
(4) A gradient centrifuge tube was prepared by adding 15 mL of gradient separation solution Ficoll Lymphoprep;
(5) The diluent was mixed with the incubated blood sample at a 1:1 ratio;
(6) The diluted blood sample was gently transferred onto the separation solution and centrifuged at 1200 g for 20 minutes;
(7) The entire supernatant after centrifugation was quickly transferred to a new centrifuge tube;
(8) The supernatant was mixed with 25 mL of diluent and centrifuged at 300 g for 10 minutes;
(9) The above steps was repeated;
(10) The T cells were resuspended in 2 mL of complete T cell medium, counted, and used for subsequent experiments.

### 3.2 Detection of NKG2D-CD3 Binding to Senescent Cells and T Lymphocytes

(1) T cells and senescent cells were resuspended in 200 µL of 1x PBS (containing 2% FBS);
(2) The resuspended cells was added with NKG2D-CD3 or control antibody at a final concentration of 100 µg/mL, mixed well, and incubated on ice for 120 minutes. Cells were subjected to vortex every 10 minutes during incubation. The cells were then centrifuged at 500 g for 5 minutes, and the supernatant was discarded;
(3) The cells were resuspended in 1 mL of 1x PBS (containing 2% FBS) and centrifuged at 500 g for 5 minutes;
(4) The step was repeated;
(5) The cells was added with anti-His antibody, mixed well, and incubated on ice for 60 minutes. Cells were subjected to vortex every 10 minutes during incubation. The cells were then centrifuged at 500 g for 5 minutes, and the supernatant was discarded;
(6) Fluorescently labeled goat anti-rabbit secondary antibody was added, and the cells were incubated at room temperature for 30 minutes, followed by centrifugation at 500 g for 5 minutes. The supernatant was discarded;
(7) The cells were resuspended in 1 mL of 1x PBS (containing 2% FBS) and centrifuged at 500 g for 5 minutes;
(8) The step 7 was repeated;
(9) The binding efficiency was detected by flow cytometry. The results are shown in Figs. 3A, 3B, and 5A, indicating that NKG2D-CD3 effectively binds to both senescent cells and T lymphocytes.

### 3.3 NKG2D-CD3 Protein Promotes the T Cell-Mediated Killing of Senescent Cells

T cells and senescent cells were co-cultured in a 96-well plate at an effector-to-target ratio of 2:1 (T cells as effector cells; senescent cells as target cells). NKG2D-CD3 or CD19-CD3 protein was added respectively and co-incubated with the cells for 8 hours (culture medium: advanced 1640 medium (Gibco) + 10% fetal bovine serum (Gibco) + 1% penicillin/streptomycin (Gibco)). The number of live cells was counted under a microscope, and the T cell killing rate was calculated. Killing efficiency = (Number of target cells in the Blank group - Number of target cells in the co-culture group) / Number of target cells in the Blank group * 100%. The results are shown in Figs. 3C, 3E, and 5B, demonstrating that compared to the control group, NKG2D-CD3 significantly promotes T cell-mediated killing of senescent cells in a dose-dependent manner.

The supernatant from the culture system was collected, and the IFN-γ concentration was detected using an ELISA kit. Statistical analysis was performed using GraphPad Prism software. The results are shown in Fig. 3D, indicating a significant increase in IFN-γ secretion after the addition of NKG2D-CD3 compared to the control group.

### Example 4 Preparation of NKG2D-CD16 and NKG2D-FC Proteins

The nucleotide sequence of the target gene with the structure shown in Fig. 6A was synthesized and cloned into the lentiviral vector pCDH-CMV-MCS-EF1-Puro through EcoR I and Swa I restriction sites. After verifying the correctness of the cloned vector through restriction enzyme digestion and sequencing, it was transformed into competent *E. coli* (Stbl3) and expanded for culture. The plasmid was then extracted using the QIAGEN endotoxin-free midi extracting kit and used for virus packaging, as described in Example 1. The packaged virus was used to infect CHO cells for protein preparation. The prepared NKG2D-CD16 and NKG2D-FC proteins were detected using an anti-His tag antibody. The results shown in Fig. 6B indicate that the protein sizes matches the expected values.

### Example 5 NKG2D-CD16 and NKG2D-FC Proteins Promote the NK Cell-Mediated Killing of Senescent Cells

### 5.1 Detection of NKG2D-CD16 and NKG2D-FC Binding to Senescent Cells and NK Cells

(1) NK cells or senescent cells were resuspended in 200 µL of 1x PBS (containing 2% FBS);
(2) The resuspended cells was added with NKG2D-CD16 and NKG2D-FC proteins at a final concentration of 100 µg/mL, mixed well, and incubated on ice for 120 minutes. Cells were subjected to vortex every 10 minutes during incubation. The cells were then centrifuged at 500 g for 5 minutes, and the supernatant was discarded;
(3) The cells were resuspended in 1 mL of 1x PBS (containing 2% FBS) and centrifuged at 500 g for 5 minutes;
(4) The step was repeated;
(5) The cells was added with anti-His antibody, mixed well, and incubated on ice for 60 minutes. Cells were subjected to vortex every 10 minutes during incubation. The cells were then centrifuged at 500 g for 5 minutes, and the supernatant was discarded;
(6) Fluorescently labeled goat anti-rabbit secondary antibody was added, and the cells were incubated at room temperature for 30 minutes, followed by centrifugation at 500 g for 5 minutes. The supernatant was discarded;
(7) The cells were resuspended in 1 mL of 1x PBS (containing 2% FBS) and centrifuged at 500 g for 5 minutes;
(8) The step 7 was repeated;
(9) The binding ratio was detected by flow cytometry. The results are shown in Figs. 7A and 7B. The results indicate that both NKG2D-CD16 and NKG2D-FC can bind to NK cells or senescent cells.

### 5.2 NKG2D-CD16 and NKG2D-FC Promotes the NK Cell-Mediated Killing of Senescent Cells

Senescent cells induced by P16 overexpression and NK cells were co-cultured in a 96-well plate at an effector-to-target ratio of 2:1 (senescent cells as target cells, NK cells as effector cells). Different concentrations of NKG2D-CD16 or NKG2D-FC protein were added and co-incubated with the cells for 8 hours. The number of live cells was counted under a microscope, and the NK cell killing rate was calculated. Killing efficiency = (Number of target cells in the Blank group - Number of target cells in the co-culture group) / Number of target cells in the Blank group. The results are shown in Fig. 7C, demonstrating that compared to the control group, both NKG2D-CD16 and NKG2D-FC significantly promote NK cell-mediated killing of senescent cells. The supernatant from the culture system was collected, and the IFN-γ concentration was detected using an ELISA kit. Statistical analysis was performed using GraphPad Prism software. The results are shown in Fig. 3D, indicating a significant increase in IFN-γ secretion after the addition of NKG2D-CD16 or NKG2D-FC compared to the control group.

All documents mentioned in the present invention are incorporated by reference herein as if each document were incorporated separately by reference. Furthermore, it should be understood that after reading the foregoing teachings of the invention, various changes or modifications may be made to the invention by those skilled in the art and that these equivalents are equally within the scope of the claims appended to this application.

## Claims

1. Use of a cell engager molecule targeting a NKG2D ligand and an immune cell for preparing a medicament used for:
(i) clearing senescent cells;
(ii) preventing and/or treating a disease associated with the accumulation of senescent cells;
(iii) delaying individual aging;
wherein the cell engager molecule comprises a first binding domain and a second binding domain,
wherein the first binding domain specifically binds to a NKG2D ligand, and the second binding domain specifically binds to a membrane protein on immune cell surface.

2. The use according to claim 1, wherein the first binding domain is an extracellular domain of NKG2D.

3. The use according to claim 1, wherein the first binding domain comprises a polypeptide with an amino acid sequence as shown in SEQ ID NO: 1, or a polypeptide with an amino acid sequence having more than 80% similarity to the sequence as shown in SEQ ID NO: 1 and capable of binding to a NKG2D ligand.

4. The use according to claim 1, wherein the second binding domain specifically binds to CD3 or CD16.

5. The use according to claim 1, wherein the second binding domain has a structure selected from the group consisting of: single domain antibody (sdAb), single chain antibody (scFv), antigen-binding fragment (Fab fragment), ligand, or a polymer thereof, or a combination thereof.

6. The use according to claim 1, wherein the second binding domain is a CD3-binding domain which is capable of specifically binding to CD3 on the surface of T lymphocyte.

7. The use according to claim 6, wherein the CD3-binding domain has an amino acid sequence as shown in SEQ ID NO: 2, or has an amino acid sequence with at least 80%, 85%, 90%, 91%, 92%, 93%, 94%, 95%, 96%, 97%, 98%, or 99% sequence homology or sequence identity to the amino acid sequence as shown in SEQ ID NO: 2.

8. The use according to claim 1, wherein the second binding domain is a CD16-binding domain which is capable of specifically binding to CD16 on the surface of natural killer cell.

9. The use according to claim 8, wherein the CD16-binding domain has an amino acid sequence as shown in SEQ ID NO: 3 or 4, or has an amino acid sequence with at least 80%, 85%, 90%, 91%, 92%, 93%, 94%, 95%, 96%, 97%, 98%, or 99% sequence homology or sequence identity to the amino acid sequence as shown in SEQ ID NO: 3 or 4.

10. The use according to claim 8, wherein the CD16-binding domain is a FC fragment of human IgG.

11. The use according to claim 10, wherein the FC fragment of human IgG has an amino acid sequence as shown in SEQ ID NO: 5, or has an amino acid sequence with at least 80%, 85%, 90%, 91%, 92%, 93%, 94%, 95%, 96%, 97%, 98%, or 99% sequence homology or sequence identity to the amino acid sequence as shown in SEQ ID NO: 5.

12. The use according to claim 1, wherein the cell engager molecule has a structure selected from the following formula (I) or (II) from N-terminus to C-terminus:
S-D₁-L₁-D₂-T (I);
or
S-D₂-L₁-D₁-T (II),
wherein,
each "-" is independently a linking peptide or peptide bond;
S is absent or a signal peptide sequence;
D₁ is the first binding domain;
Li is absent or a linker peptide;
D2 is the second binding domain;
T is absent or a marker protein.

13. The use according to claim 1, wherein the senescent cell is selected from the group consisting of: lung cell, fat cell, kidney cell, muscle cell, and a combination thereof.

14. The use according to claim 1, wherein the expression of NKG2D ligand is upregulated in the senescent cells.

15. The use according to claim 1, wherein the upregulation of NKG2D ligand expression means that the ratio of the expression level of NKG2D ligand in the senescent cells (F1) to that in normal cells (F0) (i.e., F1/F0) is ≥1.5, preferably ≥2, more preferably ≥2.5.

16. The use according to claim 1, wherein the NKG2D ligand is selected from the group consisting of: MICA, MICB, ULBP1, ULBP2, ULBP3, ULBP4, ULBP5, ULBP6, and a combination thereof.

17. The use according to claim 1, wherein the age-related disease is selected from the group consisting of: heart failure, atherosclerosis, diabetes, myocardial hypertrophy, osteoporosis, tissue/organ fibrosis, Alzheimer's disease, Parkinson's syndrome, arthritis, and a combination thereof.

18. A pharmaceutical composition which comprises:
(a) a cell engager molecule targeting NKG2D and an immune cell;
(b) other drug for clearing senescent cells in addition to (a); and
(c) a pharmaceutically acceptable carrier, diluent, or excipient.

19. A pharmaceutical composition which comprises:
(a) a cell engager molecule targeting NKG2D and an immune cell;
(b) a drug capable of upregulating the expression of NKG2D ligand; and
(c) a pharmaceutically acceptable carrier, diluent, or excipient.
